# EUROPÄISCHE PATENTSCHRIFT

(11) **EP 2 533 029 B1**
(45) Veröffentlichungstag und Bekanntmachung des Hinweises auf die Patenterteilung: **19.08.2015**
(21) Anmeldenummer: 12167611.8
(22) Anmeldetag: 11.05.2012
(51) Int. Cl.: G01N 33/18, G01M 15/10

(54) **Verfahren und Vorrichtung zur Analyse des Abgases von Brennkraftmaschinen**
Method and device for analysing the exhaust gas of internal combustion engines
Procédé et dispositif d'analyse des gaz d'échappement de moteurs à combustion interne

(30) Priorität: 10.06.2011 AT 8632011
(43) Veröffentlichungstag der Anmeldung: 12.12.2012
(73) Patentinhaber: AVL List GmbH, 8020 Graz (AT)
(72) Erfinder: Schimpl, Thomas, 8430 Leibnitz (AT); Unger, Manuel, 8510 Stainz (AT); Pointner, Volker, 8010 Graz (AT)
(74) Vertreter: Patentanwälte Pinter & Weiss OG

(56) Entgegenhaltungen:
- EP-A2- 0 822 402
- WO-A2-02/066974
- AT-U2- 6 701
- US-A- 3 593 023

## Beschreibung

Die Erfindung betrifft ein Verfahren zur Analyse des Abgases von Verbrennungskraftmaschinen, umfassend die Kühlung des Abgases vor der Analyse, die Bestimmung der Konzentration von zumindest zwei unterschiedlichen Abgaskomponenten in zumindest zwei separaten Analysestufen oder die Bestimmung der Konzentration einer einzelnen Abgaskomponente mit zumindest zwei unterschiedlichen Abkühlstufen, sowie einen Abgas-Kühler für eine Vorrichtung zur Analyse des Abgases von Verbrennungskraftmaschinen, als auch eine Vorrichtung zur Analyse des Abgases von Verbrennungskraftmaschinen, umfassend zumindest einen Kühler für das Abgas und eine Analysatoranordnung zur Bestimmung der Konzentration von zumindest zwei unterschiedlichen Abgaskomponenten in zumindest zwei separaten Analysestufen oder zur Bestimmung der Konzentration einer einzelnen Abgaskomponente mit zumindest zwei unterschiedlichen Abkühlstufen.

Zur Messung von Abgasen von Verbrennungskraftmaschinen ist eine entsprechende Aufbereitung des Abgases notwendig. Stand der Technik ist dabei die heiße Messung von Abgas bzw. die kalte Messung über einen Gaskühler. Dazu gibt es auch Geräte, die sowohl eine kalte, als auch eine heiße Messung integrieren, wie z.B. in der AT 6 701 U2 beschrieben. Dazu sind im Gerät mehrere parallele, unterschiedlich temperierte Messzweige vorgesehen. Wird Abgas kalt gemessen, wie z.B. in der WO 2002/066974 A1, so ist die Entfernung von Wasserdampf notwendig. Bei bekannten Geräten wird dazu ein Kühler eingesetzt, der das Abgas auf eine Temperatur zwischen 5 °C und 10 °C abkühlt. Damit ist für einen Wassergehalt im Abgas gesorgt, der die Messung nicht beeinflusst und die Kondensation von Wasser und höher siedenden Kohlenwasserstoffen in den Analysatoren verhindert. Der Siedepunkt der messtechnisch relevanten Abgaskomponente Stickstoffdioxid liegt bei 21,15 °C, weiters besitzt Stickstoffdioxid im Gegensatz zu den anderen Abgaskomponenten eine hohe Löslichkeit in dem im Gaskühler auskondensierten Wasser. Beides führt zu einem Verlust von Stickstoffdioxid im Gaskühler und somit zu einer geringeren Konzentration im Analysator.

Die Aufgabe der vorliegenden Erfindung war daher eine Verbesserung des Verfahrens und der Vorrichtung zur Analyse des Abgases, bei welchem eine höhere Messgenauigkeit gewährleistet ist.

Zur Lösung dieser Aufgabe ist das eingangs angegebene Verfahren erfindungsgemäß dadurch gekennzeichnet, dass zumindest eine erste Kühlung vor einem ersten Analysator und zumindest eine zweite Kühlung auf eine geringere Temperatur als jene der ersten Kühlung vor zumindest einem weiteren Analysator vorgenommen wird.

Vorteilhafterweise wird zusätzlich noch eine Vorkühlung des Abgases vor der ersten Kühlung vorgenommen.

Gemäß einer vorteilhaften Ausführungsform der Erfindung ist vorgesehen, dass die erste Kühlung auf eine Abgas-Temperatur knapp oberhalb des Taupunktes der zu bestimmenden Abgaskomponente erfolgt, vorzugsweise auf eine Temperatur zwischen 20 und 35°C. So wird beispielsweise für die Stickoxidmessung eine Kühlertemperatur gewählt, die über dem Siedepunkt von Stickstoffdioxid liegt und somit den Verlust von Stickstoffdioxid verringert.

Eine weitere bevorzugte Ausführungsform sieht vor, dass eine weitere Kühlung auf eine Abgas-Temperatur zwischen 0 und 20°C, vorzugsweise zwischen ca. 5 und 10°C, vorgenommen wird, so dass der Einfluss von Wasserdampf auf die Messung zu minimieren ist.

Zweckmäßigerweise kann als zusätzliches Merkmal bei jeder Kühlstufe zumindest eine Kondensatabscheidung, vorzugsweise zwei separate Kondensatabscheidungen vorgenommen werden.

Um auf die Durchflussmengen der eingesetzten Analysatoren Rücksicht nehmen zu können, ist eine weitere vorteilhafte Ausführungsform der Erfindung dadurch gekennzeichnet, dass vor zumindest einer der weiteren Kühlungen eine Aufteilung des Stroms des Abgases erfolgt, wobei lediglich ein Teilstrom der weiteren Kühlung zugeführt und nachfolgend analysiert wird.

Alternativ kann eine Variante vorgesehen sein, bei welcher vor zumindest einer weiteren Kühlung eine Aufteilung des Stroms des Abgases in zumindest zwei Teilströme erfolgt, wobei lediglich ein Teilstrom der weiteren Kühlung zugeführt wird, wobei aber in zumindest zwei Teilströmen die Konzentration von zumindest einer Abgaskomponente hin bestimmt wird.

Eine weitere Variante ist dadurch gekennzeichnet, dass nach jeder Aufteilung des Gasstroms lediglich ein Teilstrom weiter gekühlt wird und in zumindest zwei Teilströmen die Konzentration von zumindest einer Abgaskomponente bestimmt wird.

Um eine optimale und effiziente Kühlung für das Abgas bei Behandlung gemäß dem oben beschriebenen Verfahren zu gewährleisten, ist der Abgas-Kühler erfindungsgemäß dadurch gekennzeichnet, dass zumindest zwei mit unterschiedlichen Temperaturen betriebene Kühler als Kombinationskühler in einem gemeinsamen, vorzugsweise isolierten Gehäuse untergebracht sind. Die Anordnung der beiden Kühler in dem Modul erlaubt eine gegenseitige Kühlung und damit eine Minimierung der Kühlverluste.

Vorteilhafterweise ist für jede Kühlstufe zumindest eine separate Kondensatabscheidung vorgesehen.

Eine vorteilhafte Ausführungsform des erfindungsgemäßen Kühlers ist dadurch gekennzeichnet, dass für zumindest eine Kühlstufe zumindest eine Entnahme für zumindest einen Teilstrom vorgesehen ist.

Die eingangs beschriebene Vorrichtung ist erfindungsgemäß zur Lösung der gestellten Aufgabe dadurch gekennzeichnet, dass zumindest einem ersten Analysator eine erste Kühlstufe und zumindest einem zweiten Analysator eine zweite, auf einer niedrigen Temperatur als jene der ersten Kühlstufe betriebene weitere Kühlstufe vorgeschaltet ist.

Gemäß einer vorteilhaften Ausführungsform der Vorrichtung ist vorgesehen, dass der der weiteren Kühlstufe nachgeschaltete Analysator ein weiterer Analysator für eine weitere Abgaskomponente ist.

Vorzugsweise ist der ersten Kühlstufe ein Vorkühler vorgeschaltet.

Gemäß einer weiteren erfindungsgemäßen Ausführungsform ist die Vorrichtung weiters dadurch gekennzeichnet, dass zumindest einer der Kühler mit zumindest zwei separaten Kondensatabscheidern versehen ist, wobei vorzugsweise ein erster Kondensatabscheider nahe dem Eintritt des Abgases in den Kühler und ein zweiter Kondensatabscheider nahe dem Austritt des Abgases aus dem Kühler angeordnet sind.

Eine weitere vorteilhafte Variante sieht zusätzlich vor, dass zumindest zwischen einem Analysator und einer Kühlstufe eine Abzweigung für zumindest einen Teilstrom des Abgases vorgesehen ist.

Gegebenenfalls kann auch eine Ausführungsform dadurch gekennzeichnet sein, dass zumindest ein Analysator für zumindest einen gekühlten und zumindest einen ungekühlten Teilstrom vorgesehen ist.

Bei Verwendung von mehreren Abgaskühleren können zumindest zwei Abgaskühler für jeweils eine Kühlstufe in einem gemeinsamen Gehäuse angeordnet sein..

In der nachfolgenden Beschreibung soll die Erfindung anhand von bevorzugten Ausführungsbeispielen und unter Bezugnahme auf die beigefügten Zeichnungen näher erläutert werden.

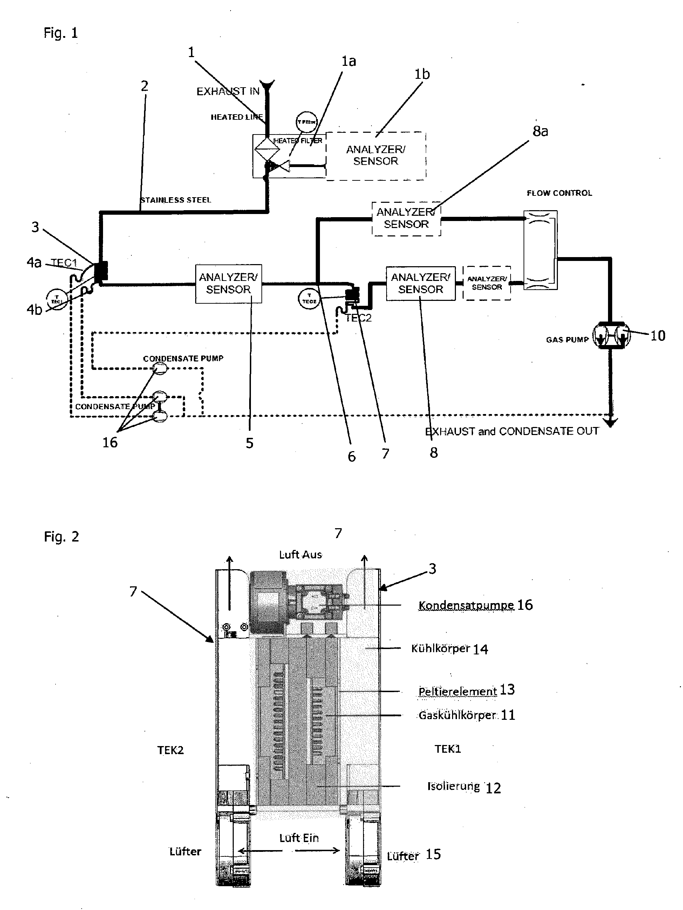

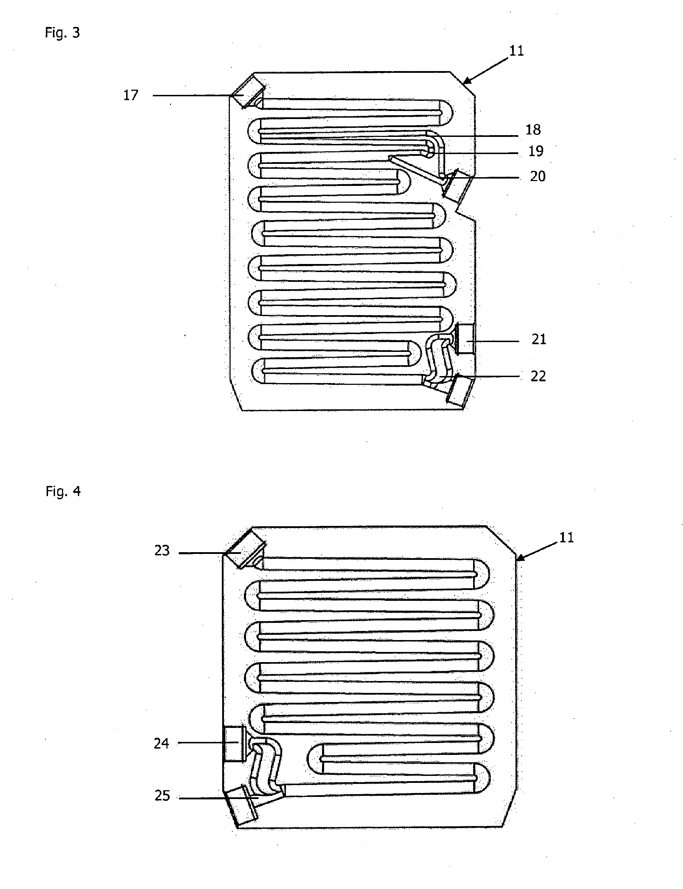

Dabei zeigt die Fig. 1 ein Flussschema für das erfindungsgemäße Verfahren und die Vorrichtung, die Fig. 2 ist eine Draufsicht auf einen Abgas-Kühler gemäß der vorliegenden Erfindung, Fig. 3 ist eine Schnittansicht der Abgasführung in der ersten Kühlstufe des Kühlers der Fig. 2 und Fig. 4 ist eine Schnittansicht der Abgasführung in der zweiten Kühlstufe des Kühlers der Fig. 2.

Entsprechend dem Flussschema der Fig. 1 wird der erfindungsgemäßen Vorrichtung zur Analyse des Abgases von Verbrennungskraftmaschinen das zu analysierende Abgas über eine beheizte Leitung 1 zugeführt und gelangt in einen beheizten Filter 1a, wo eine Partikelabscheidung und allenfalls auch Partikelmessung durch einen Analysator 1b vorgenommen wird. Über eine Stahlleitung 2, welche eine Vorkühlung des Abgases bewirkt, wird das Abgas einer ersten Kühlstufe 3 zugeführt.

Im Kühler der ersten Kühlstufe 3 erfolgt eine - nach der Vorkühlung in der Stahlleitung 2 - weitere Abkühlung des Abgases auf eine Temperatur knapp oberhalb des Taupunktes der zu bestimmenden Abgaskomponente, typischerweise auf eine Temperatur zwischen 20 und 35°C. So wird beispielsweise für die Stickoxidmessung eine Kühlertemperatur gewählt, die über dem Siedepunkt von Stickstoffdioxid von 21,15°C liegt, so dass der Verlust von Stickstoffdioxid verringert werden kann.

Vorteilhafterweise werden in der ersten Kühlstufe 3 zwei Kondensatabscheidungen über Kondensatabscheider 4a, 4b vorgenommen.

In einem nachfolgenden ersten Analysator 5 wird dann die Stickstoffmonoxid/Stickstoffdioxid-Analyse vorgenommen, wobei der Analysator 5 vorzugsweise mindestens 10 °C über dem Taupunkt des vorgeschalteten Kühlers betrieben wird..

Nach Austritt aus dem Analysator 5 gelangt das Abgas dann zu einer Kreuzungsstelle 6, wo eine Aufteilung des Stroms des Abgases erfolgt. Einer der Teilströme gelangt in eine weitere Kühlstufe 7 und nachfolgend in zumindest einen weiteren Analysator 8. Die Menge des dem Analysator 8 zugeführten Teilstroms wird auf die von diesem Analysator 8 bzw. der durch den gekühlten Teilstrom Analysatorengruppe durchströmte benötigte Menge abgestimmt. Der restliche Anteil des Abgasstroms wird als zweiter Teilstrom ungekühlt am Analysator 8 vorbeigeführt und vorzugsweise mit dem aus dem Analysator 8 austretenden Teilstrom, allenfalls nach einer Durchflusskontrolleinrichtung, wieder zusammengeführt.

Alternativ könnte auch eine Variante vorgesehen sein, bei welcher vor der weiteren Kühlstufe 7 wieder eine Aufteilung des Stroms des Abgases in zumindest zwei Teilströme erfolgt und lediglich ein Teilstrom der weiteren Kühlung 7 zugeführt wird, wobei aber in beiden Teilströmen die Konzentration von zumindest einer Abgaskomponente, gegebenenfalls der gleichen Abgaskomponente, durch parallel geschaltete Analysatoren 8, 8a bzw. Analysatorengruppen bestimmt wird. Eine weitere Variante könnte auch vorsehen, dass nach jeder Aufteilung des Gasstroms lediglich ein Teilstrom weiter gekühlt wird und in zumindest zwei Teilströmen die Konzentration von zumindest einer Abgaskomponente bestimmt wird.

Im weiteren Analysator 8 erfolgt beispielsweise die Bestimmung des Kohlenmonoxid/Kohlendioxid-Gehalts des Abgases, und für diesen Fall ist die Abkühlung des Abgases in der weiteren Kühlstufe 7 auf eine Temperatur vorzugsweise zwischen ca. 5 und 10°C vorgesehen, wodurch der Einfluss von Wasserdampf auf die Messung minimiert werden kann.. Eine Korrektur für die Wasserdampfquerempfindlichkeit kann dadurch vermieden werden.

Auch in der weiteren Kühlstufe 7 ist vorgesehen, den noch enthaltenen Wassergehalt auskondensieren zu lassen und über einen Kondensatabscheider 9 aus der Kühlstufe 7 abzuführen.

Über eine Pumpe 10 wird das Abgas durch die Vorrichtung gesaugt und nach dem letzten Analysator 8 bzw. 8a wieder abgeführt.

Wie in der Fig. 2 dargestellt ist, werden die beiden Kühlstufen 3, 7 der Fig. 1 durch zwei mit unterschiedlichen Temperaturen betriebene Kühler mit Glaskühlkörpern 11 gebildet, die für eine optimale und effiziente Kühlung als Kombinationskühler in einem gemeinsamen, vorzugsweise isolierten Gehäuse 12 untergebracht sind. Über Peltierelemente 13 werden die Kühler 11 auf die gewünschte Temperatur gebracht. Als Grundmaterial könnte vorteilhafterweise Aluminium verwendet werden, welches hartgecoatet und die Hartcoatschicht anschließend nachverdichtet ist. Auch Glas, rostfreier Stahl und PTFE könnten als Grundmaterialien Verwendung finden. Jede Kühlstufe ist mit einem weiteren Kühlkörper 14 versehen, der jeweils über einen Lüfter 15 versorgt wird. Eine Kondensatpumpe 16 pro Kühlstufe 3, 7 sorgt für die Ableitung des Kondensats aus den Kühlkörpern 11, wobei für jeden Kühlkörper 11 zumindest eine separate Kondensatabscheidung vorgesehen ist.

Die Fig. 3 zeigt die Abgasführung im Kühlkörper 11 der ersten Kühlstufe 3, in welcher an zwei Stellen eine Kondensatabscheidung vorgenommen wird. Von einem Eintritt 17 des Abgases gelangt der Gasstrom zu einer Erweiterung 18 des Gaskanales zur Verlangsamung der Strömungsgeschwindigkeit, wodurch vermieden wird, dass Tropfen anstatt abgeschieden mit dem Abgasstrom mitgerissen werden. Eine Tropfnase 19 dient zur besseren Rückhaltung und Abscheidung der Wassertropfen, die dann von einer Kondensatpumpe 16 über den ersten Kondensatauslass 20 abgesaugt werden. Damit wird vermieden, dass bereits vor und innerhalb des Kühlers verflüssigtes Wasser über die gesamte Kühlstrecke mitgenommen werden muss, was N02 Verluste reduziert und auch Energie spart. Nach weiterer Durchströmung des Kühlkörpers 11 gelangt das Abgas schließlich nach Passieren eines zweiten Abscheiders 21 mit zweitem Kondensatauslass zum Abgasauslass 22.

Die zweite Kühlstufe 7, dargestellt in Fig. 4, deren Kühlkörper 11 im gleichen Gehäuse mit der ersten Kühlstufe 3 untergebracht ist, weist ebenfalls einen Eintritt 23 für das - nun aus einem ersten Analysator 4 kommende - Abgas auf. Nach der Durchströmung des Kühlkörpers 11 dieser Kühlstufe 7 gelangt das Abgas schließlich nach Passieren des für diese Stufe einzigen Abscheiders 24 mit Kondensatauslass zum Abgasauslass 25 aus dieser Kühlstufe 7 und auch aus dem kompletten Kühlmodul.

## Patentansprüche

1. Verfahren zur Analyse des Abgases von Verbrennungskraftmaschinen, umfassend die Kühlung des Abgases vor der Analyse, die Bestimmung der Konzentration von zumindest zwei unterschiedlichen Abgaskomponenten in zumindest zwei separaten Analysatoren (5,8) oder die Bestimmung der Konzentration einer einzelnen Abgaskomponente mit zumindest zwei unterschiedlichen Abkühlstufen, **dadurch gekennzeichnet, dass** zumindest eine erste Kühlung vor einem ersten Analysator (5) und zumindest eine zweite Kühlung auf eine geringere Temperatur als jene der ersten Kühlung vor zumindest einem weiteren Analysator (8) vorgenommen wird.

2. Verfahren nach Anspruch 1, **dadurch gekennzeichnet, dass** eine Vorkühlung des Abgases vor der ersten Kühlung vorgenommen wird.

3. Verfahren nach Anspruch 1 oder 2, **dadurch gekennzeichnet, dass** die erste Kühlung auf eine Abgas-Temperatur knapp oberhalb des Taupunktes der zu bestimmenden Abgaskomponente erfolgt, vorzugsweise auf eine Temperatur zwischen 20 und 35°C.

4. Verfahren nach einem der Ansprüche 1 bis 3, **dadurch gekennzeichnet, dass** eine weitere Kühlung auf eine Abgas-Temperatur zwischen 0 und 20°C, vorzugsweise zwischen ca. 5 und 10°C, vorgenommen wird.

5. Verfahren nach einem der Ansprüche 1 bis 4, **dadurch gekennzeichnet, dass** bei jeder Kühlstufe (3,7) zumindest eine Kondensatabscheidung (4a, 4b), vorzugsweise zwei separate Kondensatabscheidungen vorgenommen werden.

6. Verfahren nach einem der Ansprüche 1 bis 5, **dadurch gekennzeichnet, dass** vor zumindest einer der weiteren Kühlungen eine Aufteilung des Stroms des Abgases erfolgt, wobei lediglich ein Teilstrom der weiteren Kühlung zugeführt und nachfolgend analysiert wird.

7. Verfahren nach einem der Ansprüche 1 bis 5, **dadurch gekennzeichnet, dass** vor zumindest einer weiteren Kühlung eine Aufteilung des Stroms des Abgases in zumindest zwei Teilströme erfolgt, wobei lediglich ein Teilstrom der weiteren Kühlung zugeführt wird, wobei aber in zumindest zwei Teilströmen die Konzentration von zumindest einer Abgaskomponente hin bestimmt wird.

8. Verfahren nach Anspruch 6, **dadurch gekennzeichnet, dass** nach jeder Aufteilung des Gasstroms lediglich ein Teilstrom weiter gekühlt wird und in zumindest zwei Teilströmen die Konzentration von zumindest einer Abgaskomponente bestimmt wird.

9. Vorrichtung zur Analyse des Abgases von Verbrennungskraftmaschinen, umfassend zumindest eine Kühlstufe (3,7) für das Abgas und einen Analysator (5,8) zur Bestimmung der Konzentration von zumindest zwei unterschiedlichen Abgaskomponenten in zumindest zwei separaten Analysestufen oder zur Bestimmung der Konzentration einer einzelnen Abgaskomponente mit zumindest zwei unterschiedlichen Abkühlstufen, **dadurch gekennzeichnet, dass** einem ersten Analysator (5) eine erster Kühlstufe (3) und einem zweiten Analysator (8) eine zweite, auf einer niedrigen Temperatur als jene der ersten Kühlstufe (3) betriebenen weitere Kühlstufe (7) vorgeschaltet ist.

10. Vorrichtung nach Anspruch 9, **dadurch gekennzeichnet, dass** die der weiteren Kühlstufe (7) nachgeschaltete Analysator (8) ein weiterer Analysator für eine weitere Abgaskomponente ist.

11. Vorrichtung nach Anspruch 9 oder 10, **dadurch gekennzeichnet, dass** der ersten Kühlstufe (3) ein Vorkühler (2) vorgeschaltet ist.

12. Vorrichtung nach einem der Ansprüche 9 bis 11, **dadurch gekennzeichnet, dass** zumindest eine der Kühlstufe (3) mit zumindest zwei separaten Kondensatabscheidern (4a, 4b) versehen ist, wobei vorzugsweise ein erster Kondensatabscheider (4a) nahe dem Eintritt des Abgases in die Kühlstufe (3) und ein zweiter Kondensatabscheider (4b) nahe dem Austritt des Abgases aus der Kühlstufe (3) angeordnet sind.

13. Vorrichtung nach einem der Ansprüche 9 bis 12, **dadurch gekennzeichnet, dass** zumindest zwischen einem Analysator (5) und einer Kühlstufe (7) eine Abzweigung (6) für zumindest einen Teilstrom des Abgases vorgesehen ist.

14. Vorrichtung nach Anspruch 13, **dadurch gekennzeichnet, dass** zumindest ein Analysator (5,8) für zumindest einen gekühlten und zumindest einen ungekühlten Teilstrom vorgesehen ist.

15. Vorrichtung nach einem der Ansprüche 9 bis 14, **dadurch gekennzeichnet, dass** zumindest zwei Abgaskühler (11, 14) für jeweils eine Kühlstufe (3 bzw. 7) in einem gemeinsamen Gehäuse (12) angeordnet sind.

16. Vorrichtung nach Anspruch 9, **dadurch gekennzeichnet, dass** zumindest zwei mit unterschiedlichen Temperaturen betriebene Kühlstufen (3, 7) als Kombinationskühler in einem gemeinsamen, vorzugsweise isolierten Gehäuse (12) untergebracht sind.

17. Vorrichtung nach Anspruch 16, **dadurch gekennzeichnet, dass** für jede Kühlstufe (3, 7) zumindest eine separate Kondensatabscheidung (19, 20, 22, 24) vorgesehen ist.

## Claims

1. A method for analyzing the exhaust gas of internal combustion engines comprising the cooling of the exhaust gas before analysis, the determination of the concentration of at least two different exhaust gas components in at least two separate analyzers (5,8) or the determination of the concentration of a single exhaust gas component with at least two different cooling stages, **characterized in that** at least one first cooling is carried out before a first analyzer (5) and at least one second cooling is carried out at a lower temperature than that of the first cooling before at least one further analyzer (8).

2. The method as claimed in claim 1, **characterized in that** a pre-cooling of the exhaust gas is carried out before the first cooling.

3. The method as claimed in claim 1 or 2, **characterized in that** the first cooling is carried out at an exhaust gas temperature just above the dew point of the exhaust gas component to be determined, preferably at a temperature of between 20 and 35°C.

4. The method as claimed in one of claims 1 to 3, **characterized in that** a further cooling to an exhaust gas temperature between 0 and 20°C, preferably between approx. 5 and 10°C, is carried out.

5. The method as claimed in one of claims 1 to 4, **characterized in that** at least one condensate separation (4a, 4b), preferably two separate condensate separations, are carried out at each cooling stage (3,7).

6. The method as claimed in one of claims 1 to 5, **characterized in that** the exhaust gas stream is divided before at least one of the further cooling stages, wherein only a partial stream is fed to the further cooling stage and subsequently analyzed.

7. The method as claimed in one of claims 1 to 5, **characterized in that** the exhaust gas stream is divided into at least two partial streams before at least one further cooling stage, wherein only one partial stream is fed to the further cooling stage, wherein however the concentration of at least one exhaust gas component is determined in at least two partial streams.

8. The method as claimed in claim 6, **characterized in that** only one partial stream is cooled further after each division of the gas stream and the concentration of at least one exhaust gas component is determined in at least two partial streams.

9. A device for analyzing the exhaust gas of internal combustion engines, comprising at least one cooling stage (3,7) for the exhaust gas and an analyzer (5,8) for determining the concentration of at least two different exhaust gas components in at least two separate analysis stages or for determining the concentration of a single exhaust gas component with at least two different cooling stages, **characterized in that** a first cooling stage (3) is connected upstream of a first analyzer (5), and a second further cooling stage (7), which is operated at a lower temperature than that of the first cooling stage (3), is connected upstream of a second analyzer (8).

10. The device as claimed in claim 9, **characterized in that** the analyzer (8) which is connected after the further cooling stage (7) is a further analyzer for a further exhaust gas component.

11. The device as claimed in claim 9 or 10, **characterized in that** a pre-cooler (2) is connected upstream of the first cooling stage (3).

12. The device as claimed in claim 9 to 11, **characterized in that** at least one of the cooling stages (3) is provided with at least two separate condensate separators (4a, 4b), wherein preferably a first condensate separator (4a) is arranged near the inlet of the exhaust gas into the cooling stage (3) and a second condensate separator (4b) is arranged near the outlet of the exhaust gas from the cooling stage (3).

13. The device as claimed in claim 9 to 12, **characterized in that** a branch (6) for at least one partial stream of the exhaust gas is provided at least between one analyzer (5) and one cooling stage (7).

14. The device as claimed in claim 13, **characterized in that** at least one analyzer (5, 8) is provided for at least one cooled and at least one uncooled partial stream.

15. The device as claimed in claim 9 to 14, **characterized in that** at least two exhaust gas coolers (11, 14) are arranged in a common housing (12) for one cooling stage (3 or 7 respectively) in each case.

16. The device as claimed in claim 9, **characterized in that** at least two cooling stages (3, 7) which are operated at different temperatures are accommodated as a combination cooler in a common, preferably insulated housing.

17. The device as claimed in claim 16, **characterized in that** at least one separate condensate separation (19, 20, 22, 24) is provided for each cooling stage (3, 7).

## Revendications

1. Procédé d'analyse des gaz d'échappement de moteurs à combustion interne, comprenant le refroidissement des gaz d'échappement avant l'analyse, la détermination de la concentration d'au moins deux composants différents des gaz d'échappement dans au moins deux analyseurs (5, 8) séparés, ou la détermination de la concentration d'un composant individuel des gaz d'échappement avec au moins deux étages de refroidissement différents, **caractérisé en ce qu'**au moins un premier refroidissement est effectué en amont d'un premier analyseur (5) et au moins un deuxième refroidissement est effectué à une température inférieure à celle du premier refroidissement en amont d'au moins un autre analyseur (8).

2. Procédé selon la revendication 1, **caractérisé en ce que** le pré-refroidissement des gaz d'échappement est effectué avant le premier refroidissement.

3. Procédé selon la revendication 1 ou 2, **caractérisé en ce que** le premier refroidissement est effectué à une température de gaz d'échappement juste au dessus du point de rosée du composant des gaz d'échappement à déterminer, de préférence, à une température comprise entre 20 et 35°C

4. Procédé selon l'une des revendications 1 à 3, **caractérisé en ce qu'**un refroidissement supplémentaire est effectué à une température de gaz d'échappement comprise entre 0 et 20°C, de préférence entre environ 5 et 10°C.

5. Procédé selon l'une des revendications 1 à 4, **caractérisé en ce qu'**au moins une séparation de condensat (4a, 4b), de préférence deux dépôts de condensats séparés, est effectuée à chaque étage de refroidissement (3, 7).

6. Procédé selon l'une des revendications 1 à 5, **caractérisé en ce qu'**une division du flux de gaz d'échappement est effectuée avant l'un au moins des autres refroidissements, un flux partiel seulement étant amené à l'autre refroidissement puis analysé.

7. Procédé selon l'une des revendications 1 à 5, **caractérisé en ce qu'**une division du flux de gaz d'échappement en au moins deux flux partiels est effectuée avant au moins un autre refroidissement, un flux partiel seulement étant amené à l'autre refroidissement, mais la concentration d'au moins un composant des gaz d'échappement étant déterminéee dans au moins deux courants partiels,.

8. Procédé selon la revendication 6, **caractérisé en ce qu'**un flux partiel seulement est en outre refroidi après chaque division du flux de gaz et la concentration d'au moins un composant de gaz d'échappement étant déterminée dans au moins deux flux partiels.

9. Dispositif d'analyse des gaz d'échappement de moteurs à combustion interne, comprenant au moins un étage de refroidissement (3, 7) destiné aux gaz d'échappement et un analyseur (5, 8) servant à déterminer la concentration d'au moins deux composants de gaz d'échappement différents dans au moins deux étages d'analyse séparées ou servant à déterminer la concentration d'un composant particulier de gaz d'échappement avec au moins deux étages de refroidissement différents, **caractérisé en ce qu'**un premier étage de refroidissement (3) est monté en amont d'un premier analyseur (5) et un second étage de refroidissement (7) supplémentaire, utilisé à une température inférieure à celle du premier étage de refroidissement (3), est monté en amont d'un second analyseur (8).

10. Dispositif selon la revendication 9, **caractérisé en ce que** l'analyseur (8), monté en aval de l'autre étage de refroidissement (7), est un analyseur supplémentaire destiné à un autre composant de gaz d'échappement.

11. Dispositif selon la revendication 9 ou 10, **caractérisé en ce qu'**un pré-refroidisseur (2) est monté en amont du premier étage de refroidissement (3).

12. Dispositif selon l'une des revendications 9 à 11, **caractérisé en ce que** l'un au moins des étages de refroidissement (3) est équipé d'au moins deux séparateurs de condensats (4a, 4b) séparés, de préférence un premier séparateur de condensat (4a) étant disposé à proximité de l'entrée des gaz d'échappement dans l'étage de refroidissement (3) et un deuxième séparateur de condensat (4b) est disposé à proximité de la sortie des gaz d'échappement de l'étage de refroidissement (3).

13. Dispositif selon l'une des revendications 9 à 12, **caractérisé en ce qu'**une dérivation (6) destinée à au moins un flux partiel de gaz d'échappement est prévue entre au moins un analyseur (5) et un étage de refroidissement (7).

14. Dispositif selon la revendication 13, **caractérisé en ce qu'**il est prévu au moins un analyseur (5, 8) pour au moins un flux partiel refroidi et pour au moins un flux partiel non refroidi.

15. Dispositif selon l'une des revendications 9 à 14, **caractérisé en ce qu'**au moins deux refroidisseurs de gaz d'échappement (11, 14) destinés respectivement à un étage de refroidissement (3 resp. 7) sont disposés dans un boîtier commun (12).

16. Dispositif selon la revendication 9, **caractérisé en ce qu'**au moins deux étages de refroidissement (3, 7), fonctionnant à des températures différentes, sont placés en tant que refroidisseur combiné dans un boîtier commun, de préférence un boîtier isolé (12).

17. Dispositif selon la revendication 16, **caractérisé en ce qu'**au moins une séparation de condensat (19, 20, 22, 24) distincte est prévue pour chaque étage de refroidissement (3, 7).
